# EUROPEAN PATENT SPECIFICATION

(11) **EP 0 246 307 B1**
(45) Date of publication and mention of the grant of the patent: **02.02.1994**
(21) Application number: 86907171.2
(22) Date of filing: 13.11.1986
(51) Int. Cl.: C07K 7/04, A23J 1/14, A61K 35/78, C12N 15/00, C12N 9/12, C07H 15/12

(54) **CYS CODON-MODIFIED DNA**
DURCH CYS-KODON MODIFIZIERTE DNS
ADN MODIFIE PAR UN CODON CYS

(30) Priority: 13.11.1985 US 798163
(43) Date of publication of application: 25.11.1987
(73) Proprietor: THE UNIVERSITY HOSPITAL, Boston, Massachusetts 02118-2393 (US)
(72) Inventor: THE UNIVERSITY HOSPITAL, Boston, Massachusetts 02118-2393 (US)
(74) Representative: Sheard, Andrew Gregory
(86) International application number: US8602444
(87) International publication number: WO8702987

(56) References cited:
- WO-A-83/03971
- WO-A-84/00299
- US-A- 4 468 382
- SCIENCE, vol. 220, no. 4596, 29 April 1983; D. LEONG et al., pp. 515-517#
- PROCEEDINGS OF THE NATL. ACADEMY OF SCIENCE USA, vol. 83, no. 21, November 1986, Washington, DC (US); J.R. MURPHY, pp. 8258-8262#
- EUR. J. BIOCHEM., vol. 134, August 1983, Berlin (DE); MAASSEN et al., pp. 327-330#
- THE EMBO JOURNAL, vol. 4, 1985, Oxford (GB); JACOB et al., pp. 3339-3343#
- JOURNAL OF CELLULAR BIOCHEMISTRY, vol. 20, 20 August 1982, New York, NY (US); HERSCHMANN et al., pp. 163-176#
- JOURNAL OF BIOLOGICAL CHEMISTRY, vol. 258, 10 February 1983, Baltimore, MD (US); BACHA et al., pp. 1565-1570#
- V.J. HARUBY et al., "Peptides, Structures and Function: Proceedings of the 8th American Peptide Symp.", 1983, Pierce Chemical Co., Rockford, IL (US); pp. 837-852#
- PROCEEDINGS OF THE NATL. ACADEMY OF SCIENCE USA, vol. 80, Novemver 1983, Washington, DC (US); GREENFIELD et al., pp. 6853-6857#
- JOURNAL OF BIOLOGICAL CHEMISTRY, vol. 256, 10 June 1981, Baltimore, MD (US); ROTH et al., pp. 5350-5354#

## Description

This invention was made in part with government funding, and the government has certain rights in the invention.

This invention relates to the use of recombinant DNA techniques to make analogs of toxin molecules, and to the use of such molecules to treat medical disorders.

The literature contains a number of examples of hybrid molecules containing a specific binding ligand-portion and a toxin portion (e.g., ricin or diphtheria toxin); the ligand targets the toxin to an unwanted class of cells, sparing healthy cells, to which the ligand fails to bind.

For example, Bacha et al. U.S. Pat. No. 4,468,382 (hereby incorporated by reference) describes hybrid molecules made by derivatizing a neuropeptide hormone (e.g., thyrotropin releasing hormone) and an enzymically active fragment of diphtheria toxin using sulfur-containing groups and then reacting the derivatized molecules to join them via a disulfide bond. One disadvantage of this approach is that the site of derivatization on both molecules cannot be precisely controlled, so that the final product is heterogeneous, containing some molecules in which derivatization and coupling has impaired the toxicity or binding capacity of the hybrid molecule.

An approach which deals with this problem of heterogenicity is described in Murphy PCT International Publication No. WO/83/03971 (hereby incorporated by reference). The Murphy application describes hybrid proteins encoded by genes encoding both the toxin and the specific binding portion of the hybrid protein. This approach, of course, can be used only for DNA-encoded peptide ligands.

### Summary of the Invention

The present invention provides toxin molecules which can be linked to any specific-binding ligand, whether or not it is a peptide, at a position which is predeterminedly the same for every toxin molecule.

The invention generally features, in one aspect, a DNA sequence encoding a fragment of a toxin molecule which is large enough to exhibit cytotoxic activity and small enough to fail to exhibit generalized eucaryotic cell binding; the DNA sequence includes a non-naturally occuring cysteine codon, preferably located such that the fragment encoded by the DNA sequence, when linked to a cell-specific ligand via the cysteine residue encoded by the cysteine codon, exhibits cytotoxic enzymic activity.

In preferred embodiments, the toxin is diphtheria toxin, ricin, or abrin; the cysteine codon is introduced at the C-terminal-encoding end of the toxin-encoding DNA sequence or within 100 base pairs thereof; the ligand is a peptide hormone, a proteinaceous growth factor (preferably Interleukin I, Interleukin II, Interleukin III, or B-cell growth factor), an antibody, or a steroid hormone (e.g., estradiol).

In another aspect, the invention features a specific binding peptide ligand, and DNA sequences coding therefor, which can bind to any reactive sulfur group-containing toxin molecule in a predetermined and consistent manner. The DNA sequence of this aspect of the invention encodes a fragment of a ligand (preferably one of those listed above) which is large enough to exhibit specific cell binding; the gene includes a non-naturally occuring cysteine codon, preferably located such that the fragment encoded by the DNA sequence, when linked to a toxin via the cysteine residue encoded by the cysteine codon, exhibits specific cell binding.

The invention also features the hybrid molecules made using the Cys-modified toxins and ligands of the invention, as well as the methods for making such hybrid molecules.

Other features and advantages of the invention will be apparent from the following description of the preferred embodiments thereof, and from the claims.

### Description of the Preferred Embodiments

The drawings will first briefly be described.

### Drawings

Fig. 1 is a partial restriction map of a DNA fragment of the invention, in plasmid pABC1508.

Fig. 2 is a diagrammatic representation of the diphtheria toxin molecule.

Fig. 3 is a restriction map showing the location and orientation of the diphtheria tox gene on the 3.9 BamHI-I restriction fragment of corynephage beta^{tox}.

Figs. 4-5 are diagrammatic representations of the steps involved in the construction of pABC1508.

Fig. 6 is a diagrammatic representation of a plasmid, pMSH53 containing α -MSH-encoding DNA.

Fig. 7 is the nucleotide sequence of the tox²²⁸ allele and flanking regions, with amino acid residues shown above nucleotides; the tox²²⁸ allele is the same as the wild-type tox allele except for several mutations, notably the presence on the tox²²⁸ allele of an NruI site (Fig. 7 was adapted from Fig. 1 of Kaczorek et al. (1983) Science 221, 855).

### Tox Gene

The tox gene, and the diphtheria toxin molecule it encodes, will now briefly be described.

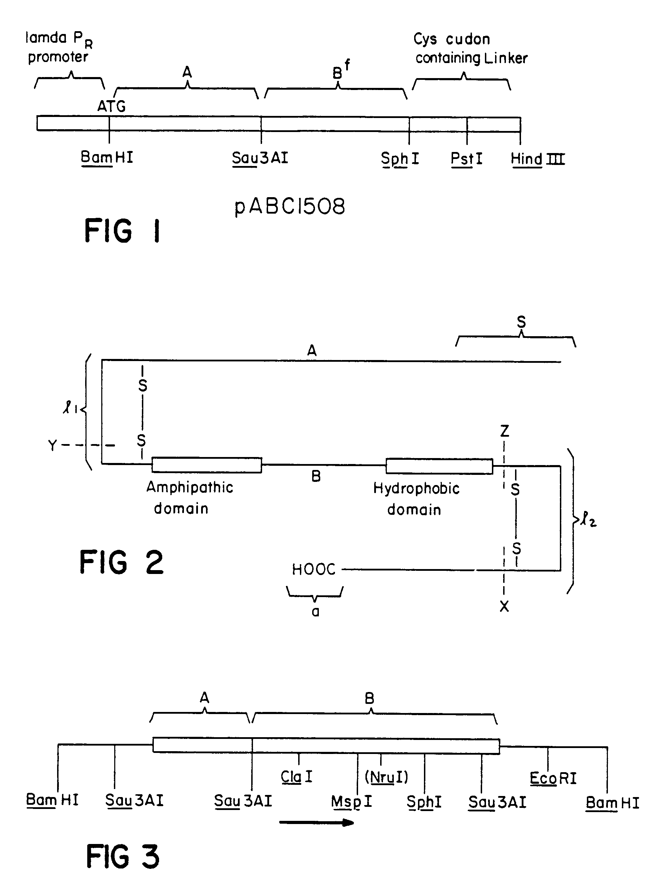

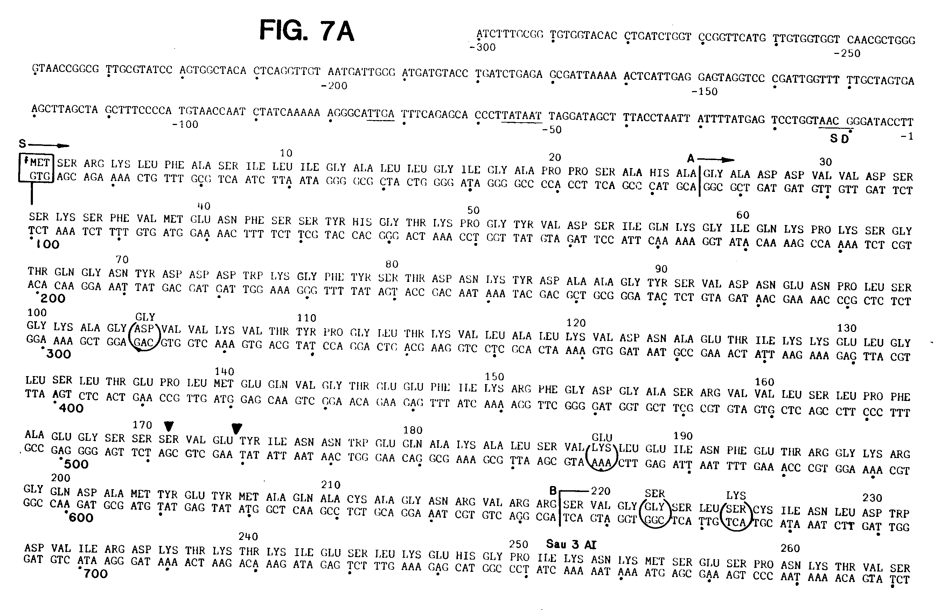

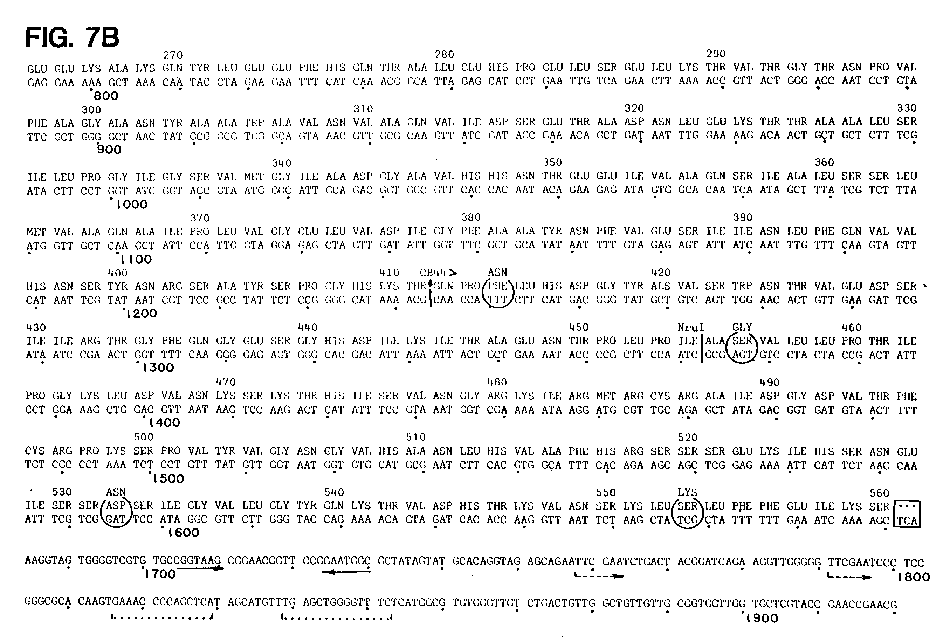

Figs. 2 and 3, illustrate, respectively, the diphtheria toxin molecule and the diphtheria tox gene, located on the 3.9 kb BamHI restriction fragment of corynephage beta^{tox}. Fig. 7 gives the sequence of the tox 278 allele.

Referring to Fig. 2, the diphtheria toxin molecule consists of several functional "domains" which can be characterized, starting at the amino terminal end of the molecule, as a hydrophobic signal sequence; enzymically active Fragment A, the fourteen amino acid exposed protease sensitive disulfide loop (DSL) 1₁, containing a cleavage domain; Fragment B, which includes the lipid associating regions, e.g., a hydrophilic amphipathic domain and a hydrophobic domain; DSL 1₂; and carboxy terminal end a. DSL 1₁ contains three arginine residues; the Sau3A1 site between Fragment A and Fragment B (see Fig. 3) is at a position on the diphtheria toxin gene corresponding to the arginine residue farthest downstream of the three.

The process by which diphtheria toxin intoxicates sensitive eukaryotic cells involves at least the following steps: (i) diphtheria toxin binds to specific receptors on the surface of a sensitive cell; (ii) while bound to its receptor, the toxin molecule is internalized in an endocytic vesicle; (iii) either prior to internalization, or within the endocytic vesicle, the toxin molecule may be cleaved (or processed) at a site in the region of 47,000 daltons from the N-terminal end; (iv) as the pH of the endocytic vesicle decreases to below 5.5, the processed form of toxin, while still bound to its receptor, spontaneously inserts into the endosomal membrane; (v) once embedded in the membrane, the lipid associating regions form a pore; (vi) a proteolytic cleavage in 1₁, between Fragment A and B, occurs; (vii) thereafter, Fragment A, or a polypeptide containing Fragment A, is released into the cytosol; (viii) the catalytic activity of Fragment A, i.e., the nicotinamide adenine dinucleotide-dependent adenosine diphosphate ribosylation of Elongation Factor 2, causes the death of the intoxicated cell. It is apparent that a single molecule of Fragment A introduced into the cytosol is sufficient to kill a cell.

### Modified Tox Gene

Referring to Fig. 1, there is shown the region of plasmid pABC1508 which encodes a peptide of the invention.

The DNA region shown in Fig. 1 includes the lambda P_{R} promoter (substituted for the promoter naturally associated with the tox gene); an ATG initiation site; a DNA sequence encoding enzymically active Fragment A of diphtheria toxin; a portion of the DNA region encoding Fragment B of diphtheria toxin; and a linker containing a Cys codon.

Referring to Figs. 1-3, the portion of the diphtheria tox gene used to make a DNA sequence of the invention includes the region encoding enzymically active Fragment A (and preferably the hydrophobic leader sequence preceding Fragment A), and a portion of the Fragment B-encoding region at least as long as that ending at the MspI site. As shown in Fig. 3, the Fragment A-encoding region (including the leader sequence) begins just downstream from a convenient Sau3AI site. The MspI site is the approximate location of the end of the region of the tox gene which encodes cross reacting material 45 (CRM 45), described in Bacha et al., id. This portion of the diphtheria toxin molecule contains the lipid associating regions of Fragment B, but does not contain 1₂, and is represented in Fig. 2 as the portion of Fragment B between y and z. The Fragment B-encoding region employed can end anywhere beyond MspI, up to the SphI site. If the SphI site is used, 1₂ is included, and the portion of Fragment B is that between y and x in Fig. 3. As previously mentioned, any region ending between MspI and SphI can be used; one example is the region ending at the position of NruI, which, like the region ending at MspI, encodes a Fragment which does not contain 1₂. Any region shorter than one ending at MspI should not be used because such a fragment will not include enough of the transverse lipid associating region and thus not bring about pore formation, which is necessary for toxicity. Portions of Fragment B encoded by regions ending downstream of SphI should not be used to avoid including the diphtheria toxin receptor binding domain. (NruI is not found on the wild-type tox allele, but only on the mutant tox²²⁸ allele, described in Kaczorek et al. (1983) Science 221, 855.)

In the illustrated DNA construct (Fig. 1) the Cys codon is located at the C-terminal end of the tox-encoding DNA sequence. This location ensures that the linker containing the Cys codon will not interfere with the enzymic activity of Fragment A. Other locations in the molecule which are downstream from the Fragment A encoding region can also be used, i.e., the Cys codon-containing linker can be inserted anywhere in the Fragment B-encoding region.

Other toxins which are DNA- encoded amino acid chains can be used, in addition to diphtheria toxin; examples are ricin and the plant toxin abrin. Ligands

The specific-binding ligands used in the invention can consist of an entire ligand, or a portion of a ligand which includes the entire binding domain of the ligand, or an effective portion of the binding domain. It is most desirable to include all or most of the binding domain of the ligand molecule. In the case of alpha-MSH, a small peptide of thirteen amino acids, or beta-MSH, which contains seventeen amino acids, the portion of the molecule consisting of nine amino acids at the carboxy terminal end of the molecule, which contains the receptor-specific binding domain, can be used, or, more preferably, the entire molecule can be used. It is most preferred that at least a portion of the ligand not involved in cell binding be included, so that derivatization can be carried out in this nonbinding portion, minimizing the chance that derivatization will interfere with binding. For example, derivatization of alpha-MSH is preferably carried out at or near the N-terminal end of the molecule, because the C-terminal end contains the specific binding domain.

The regions within cell-specific ligands in which the binding domain is located are now known for a number of such ligands. Furthermore, recent advances in solid phase polypeptide synthesis can enable those skilled in this technology to determine the binding domain of practically any peptide ligand, by synthesizing various fragments of the ligand, and testing them for the ability to bind the class of cells to be killed.

The specific class of cells which are bound and killed by the hybrids of the invention is determined by the specific ligand which imparts the binding domain of the hybrid molecule. Any cell-specific ligand can be used which has a binding domain which is specific for a particular class of cells which are to be killed. Polypeptide hormones are useful such ligands. Hybrid proteins made using alpha- or beta-MSH, for example, can selectively bind to melanocytes, rendering the hybrids useful in the treatment of primary melanoma and metastic melanoma loci. Other specific-binding ligands which can be used include the proteinaceous growth factors interleukin I, interleukin II, interleukin III, and B-cell growth factor. Interleukin II is of particular importance because of its role in allergic reactions and autoimmune diseases such as Systemic Lupus Erythmatosis (SLE) , involving activated T cells. Hybrids made using B-cell growth factor can be used as immunosuppressant reagents which kill proliferating B-cells, which bear B-cell growth factor receptors, and which are involved in hypersensitivity reactions and organ rejection.

The other major class of specific binding proteins are antibodies. The antibodies most useful are those against tumors; such antibodies (generally monoclonal) are already well-known targeting agents used in conjunction with covalently bound cytotoxins. In the present invention, the anti-tumor antibodies (preferably not the whole antibody, but just the Fab portion) are those which recognize a surface determinant on the tumor cells and are internalized in those cells via receptor-mediated endocytosis; antibodies which are capped and shed will not be as effective.

Other useful polypeptide ligands having cell-specific binding domains are somotostatin, follicle stimulating hormone (specific for ovarian cells); luteinizing hormone (specific for ovarian cells); thyroid stimulating hormone (specific for thyroid cells); vasopressin (specific for uterine cells, as well as bladder and intestinal cells) ; prolactin (specific for breast cells); and growth hormone (specific for certain bones cells).

Peptide hormones must be derivatized with a sulfhydryl group reactive with the Cys of the toxin molecule. This can be carried out by inserting a Cys codon-containing linker into an appropriate location in a DNA sequence encoding the hormone, in a manner analogous to that described below for the tox gene. Alternatively, a sulfhydryl group, either by itself or as part of a Cys residue, can be introduced using solid phase peptide synthesis techniques. For example, the introduction of sulfhydryl groups into peptides is described in Hiskey (1981) Peptides 3, 137. Derivatization can also be carried out according to the method described for the derivatization of the peptide hormone thyrotropin releasing hormone in Bacha et al. U.S. Pat. No. 4,468,382, id. Similarly, proteins can be derivatized at the DNA or protein chemistry level. The introduction of sulfhydryl groups into proteins is described in Maasen et al. (1983) Eur. J. Biochem. 134, 2, 32.

The major class of non-peptide specific binding ligands useful in the invention are the steroid hormones. One example is estrogen and estrogen derivatives such as estradiol; these are currently used in the treatment of prostate carcinoma and post-menopausal mammary carcinoma. Hybrids containing these hormones, or analogs thereof, can be used in the same or smaller dosages, to treat the same diseases.
The derivatization of steroid hormones can be carried out using standard techniques, such as those described in Ikagawa et al. (1981) J. Org. Chem. 46, 18, 3747. For example, the steroid hormone 17-beta-estradiol can be derivatized, substituting an SH group for a hydroxyl group, to yield

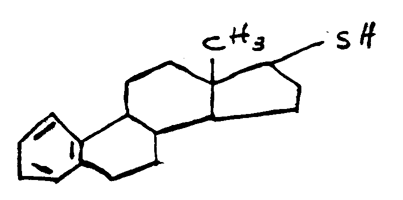

### Gene Construction

Generally, plasmids are manipulated according to standard techniques. Plasmid DNA is digested with restriction endonucleases as recommended by the manufacturer (e.g., New England Biolabs, Beverly, Mass.). Restriction fragments are electrophoresed in 1% horizontal agarose gels for 30-60 minutes at 80-100 V in TBE (89 mM boric acid, 89 mM Trizma base [Sigma Chemical Co., St. Louis, Mo.], 2.5 mM EDTA, pH 7.0) in the presence of 200 ng/ml ethidium bromide. Small DNA fragments are electrophoresed in 8% vertical polyacrylamide gels at 100 V for 2-5 hours, and stained with ethidium bromide. Gels are photographed on an ultraviolet transilluminator on Polaroid type 667 film using a red filter.

Plasmid pABC508 was constructed by fusing two pieces of DNA, one encoding Fragment A, and the other encoding part of Fragment B, to which a Cys codon-containing linker had been attached.

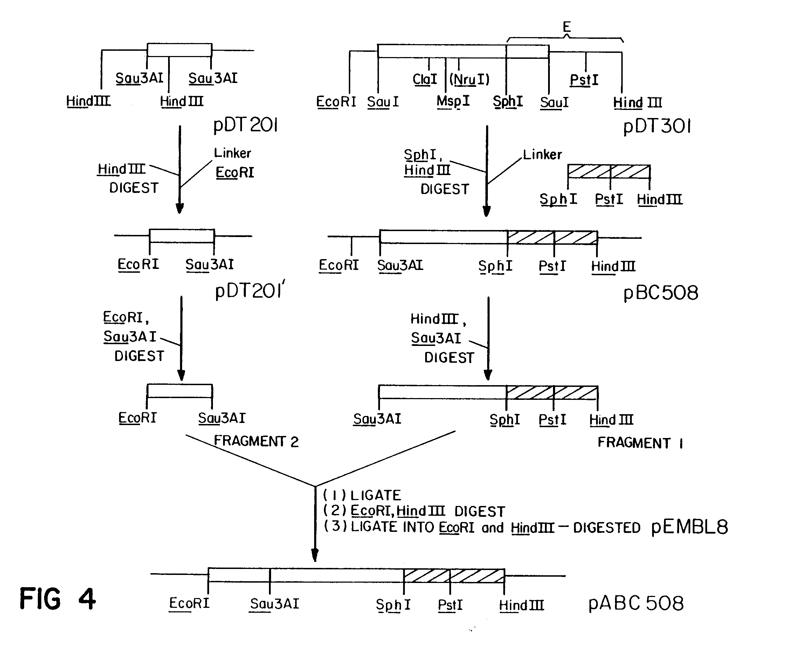

Referring to Fig. 4, this fusion was constructed from two plasmids, pDT201, which contains the fragment A-encoding region, and pDT301, which contains most of the fragment B-encoding region of the diphtheria toxin gene. The construction of each of these pieces of DNA is described below.

Plasmid pDT301 was constructed by cutting out of the tox allele a Sau3AI-1 sequence encoding all but the C-terminal 17 amino acids of Fragment B. This sequence, which carries the restriction endonuclease sites ClaI, MspI, and SphI, was inserted into the BamHI site of plasmid pUC8 (described in Viera et al. (1982) Gene 19, 259) to yield pDT301. Plasmid pDT201 contains the Fragment A-encoding Sau3AI-2 sequence (Fig. 3) (see Leong et al. (1983) Science 220, 515). (pDT301 and pDT201, in E. coli, have been deposited in the American Type Culture Collection, Rockville, MD and given ATCC Accession Nos., respectively, 39360 and 39359. Applicant's licensee, Seragen, Inc., acknowledges its responsibility to replace these cultures should they die before the end of the term of a patent issued hereon, and its responsibility to notify the ATCC of the issuance of such a patent, at which time the deposits will be made available to the public for a period of at least 30 years after the date of deposit. Until that time the deposits will be made available to the Commissioner of Patents under the terms of 37 CFR §1.14 and 35 USC §112.)

Still referring to Fig. 4, plasmid pDT301 was modified by the addition of a Cys codon-containing linker as follows. A synthetic linker was constructed on a controlled pore glass solid phase support in a 380A DNA Synthesizer (Applied Biosystems, Inc., Foster City, CA) by hybridization of 21-mer and 29-mer oligonucleotides through a 21 bp homologous core, leaving a 4bp 1/2 SphI and 1/2 HindIII single-stranded sequence on each end. This linker has the sequence 
AlaAlaAlaCysStp
5'-CGGCTGCAGCATGTTAGTAGA-3'
3'-GTACGCCGACGTCGTACAATGATCTTCGA-5'
1/2 Sphl PstI C 1/2 HindIII
The linker encodes three alanine residues, and contains a Cys codon (TGT) and a Stop codon (TAG). (This design not only allows for the expression of a Cys-containing peptide according to the present invention, but also could allow for the insertion at the PstI site of a gene encoding a specific binding ligand.)

As shown in Fig. 4, pDT301 was digested with SphI and HindIII to remove the DNA region designed "E" in Fig. 4, and the Cys codon-containing linker was then ligated into the plasmid at the SphI, HindIII sites to give plasmid pBC508. pBC508 was then cut with HindIII and Sau3AI to give Fragment 1.

Still referring to Fig. 4, plasmid pDT201 was digested with HindIII and the single-stranded ends filled in with DNA polymerase I (Klenow fragment). The resulting blunt ends were ligated to the double-stranded EcoRI linkers 
CCTTAAGG
GGAATTCC
(New England Biolabs, Beverly, MA) to give pDT201', which was then cut with EcoRI and Sau3A to give Fragment 2.

Fragments 1 and 2 were mixed in equimolar concentrations ligated together, according to standard procedures, and the mixture was then digested with EcoRI and HindIII. The digested mixture was then ligated into the EcoRI and HindIII digested pEMBL8 (Dente et al. (1983) Nucleic Acid Res. 11, 1645), which contains unique EcoRI and HindIII sites, to give pABC508. Plasmid pABC508 can be transformed into a suitable host, e.g., E. coli, as described below, to produce Cys-modified toxin molecules.

Alternatively, the naturally occurring tox promoter can be replaced with a different promoter, as follows.

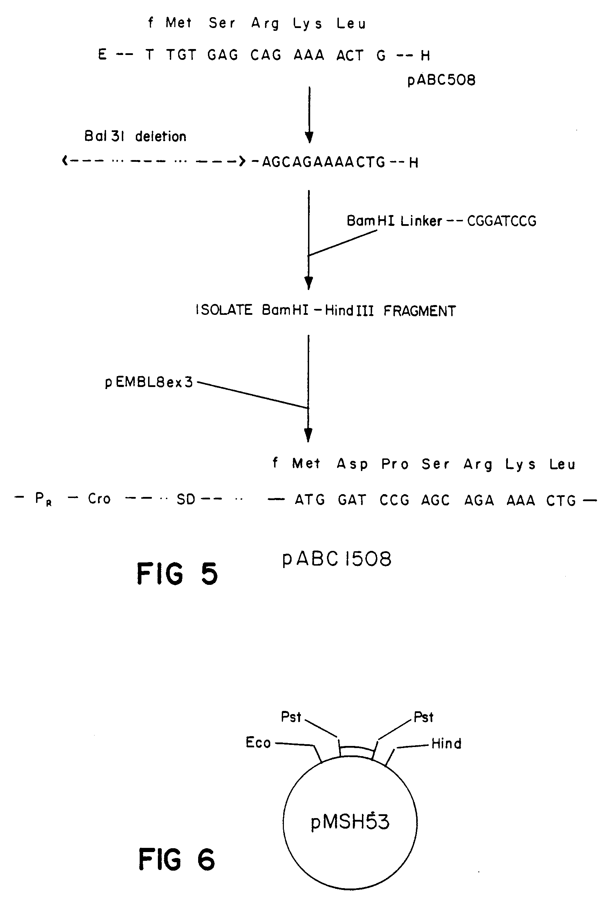

The lambda P_{R} promoter is contained in the expression vector pEMBL8ex3 (Dente et al., id). Referring to Fig. 5, the DNA sequence around the initiation site of the tox gene is shown, as are the corresponding amino acids. pABC508 was cut with EcoRI and then treated with Bal31 for a period of 10-15 minutes at 37°C with one unit of enzyme per microgram of DNA. The resulting mixture of DNA fragments was ligated to the BamHI linkers 
CCTAGGCC,
GGATCCGG
transformed into E. coli HB101 (Bethesda Laboratories, Gaithersburg, MD), and the DNA sequence of the region encoding the 5' end of tox, and the sequence of 30 of the resulting clones determined. One clone, containing the DNA sequence shown in Fig. 5, was purified and the BamHI-HindIII fragment isolated and inserted into pEMBL8ex3 which had been cut with BamHI and HindIII. The resulting plasmid, pABC1508, contains the lambda P_{R} promoter and an ATG translational start codon. An extra asparagine and proline residue are inserted during this process. In Fig. 5, Cro represents the Cro gene of lambda, and SD represents the Shine-Dalgarno sequence. The lambda P_{R} promoter can be regulated by the lambda cI gene. In this example the mutant cI₈₅₇ temperature-sensitive repressor gene is used such that the P_{R} promoter is inactive at 30^{o}C and active at 37^{o}C.

pABC1508 was transformed, using conventional techniques (e.g., as described in Maniatis et al. (1984) Molecular Cloning: A Laboratory Manual, Cold Spring Harbor, N.Y.), into E. coli HB101 (others, e.g., E. coli JM101 or 5Y327, can also be used) and the expression of the diphtheria tox gene products analyzed. The introduction of the positively charged asparagine residue in the tox signal sequence does not affect the export of the tox polypeptides into the periplasmic compartment of the recombinant host.

E. coli cells transformed with vectors containing Cys-modified toxin-excoding DNA are grown under standard culture conditions, e.g., in Luria Broth containing, per liter, 10 g tryptone, 10 g NaCl, and 5 g yeast extract, and supplemented with 100 g/ml ampicillin. The diphtheria toxin-related molecules, which are exported to the periplasmic space, are purified from periplasmic extracts. Periplasmic extracts are prepared from cells grown in 9.5 liter volumes at 37°C to an A₅₉₀ of approximately 1.0. If the natural tox promoter has been replaced with temperature sensitive cI857 regulatory sequences under the control of the temperature-sensitive cI₈₅₇ gene, as described herein, cells are grown at 30^{o}C, and expression is induced by increasing the incubation temperature to 42°C for 15 min. The culture is then grown at 40°C for an additional hour. In either instance, the culture is concentrated to approximately 1 liter by filtration through 0.45 µ membranes (Pellicon system, Millipore Corp., Bedford, Mass.) and chilled to 4°C. Bacteria are harvested by centrifugation, resuspended in ice cold 20% sucrose, 30mM Tris-HCl, 1 mM EDTA, pH 7.5, and then digested with lysozyme (750 g/ml final concentration) for 30 minutes. Spheroplasts are removed by centrifugation, 2 mg p-amidinophenylmethylsulfonylfluoride (p-APMSF, Calbiochem, San Diego, Calif.) is added, and the periplasmic extract is sterilized by filtration through 0.2 µ membranes.

The Cys-modified toxin-related molecules are then purified by chromatography on Phenyl-Sepharose (Pharmacio Fine Chemicals, Piscataway, N.J.) and DEAE-cellulose essentially as described by Rappuoli et al. (1985) Biotechnology, p. 165. Periplasmic extracts are dialysed against 10mM sodium phosphate (pH 7.2) buffer, and ammonium sulfate added to 13% (w/v). The crude extracts are then applied to a Phenyl-Sepharose column equilibrated with 10 mM phosphate buffer containing 13% ammonium sulfate. The modified toxin is eluted and dialysed against 10 mM phosphate buffer, and then applied to DEAE-cellulose column. After washing with phosphate buffer, the DEAE-cellulose column is developed with a linear NaCl gradient in phosphate buffer.

The modified toxin is then applied to an anti-diphtheria toxin immunoaffinity column, containing antibody made as described in Zucker et al. (1984) Molecular Immunol. 21, 785. Following extensive wahsing, the modified toxin is eluted with 4 M guanidine hydrochloride, and immediately dialysed against phosphate buffer. The purified modified toxin is then concentrated to approximately 100 g/ml by placing the dialysis bag in dry Sephadex G-200. All purification procedures are carried out at 4^{o}C, and the modified toxin is stored in small aliquots at -76^{o}C until used.

### Specific Binding Ligand: Alpha-MSH

Referring to Fig. 6, there is shown plasmid pMSH53, which contains a DNA insert encoding alpha-MSH. pMSH53 was made by inserting into pUC8 an alpha-MSH-encoding sequence having a 1/2 PstI site at each end: 
5' GCAAGTTATAGCATGGAACATTTTAGATGGGGAAAACCTGTATAGCTGCA 3'
3' ACGTCGTTCAATATCGTACCTTGTAAAATCTACCCCTTTTGGACATATCG 5'
1/2 PstI 1/2 PstI
Plasmid pMSH53 can be used to create, using conventional methods, an expression vector for the production of alpha-MSH in cultured bacterial cells e.g., E. coli. Further, prior to such expression, a Cys-containing linker can be fused to or near the N-terminal end of the alpha-MSH-encoding sequence, in a manner analogous to the method described above for the tox gene, to produce a modified alpha-MSH containing an N-terminal Cys capable of reacting with the added Cys of the diphtheria toxin fragment. Alternatively, the Cys-containing MSH molecule can be chemically linked to any toxin molecule on which a reactive sulfhydryl group is present or has been added post-translationally, i.e., at the protein chemistry, not the DNA, level.

Rather than making alpha-MSH using recombinant DNA techniques as described above, alpha-MSH can be purified from biological sources, or obtained commercially (e.g., from Sigma Chemical Co., St. Louis, MO).

### Chemical Linkage

After an available Cys has been added to the ligand by genetic engineering techniques, or if the ligand contains an available reactive Cys or other sulfur-containing group, the toxin and ligand are coupled by reducing both compounds and mixing toxin and ligand, in a ratio of about 1:5 to 1:20, and the disulfide reaction is allowed to proceed at room temperature to completion (generally, 20 to 30 minutes). The mixture is then dialyzed extensively against phosphate buffered saline to remove unreacted ligand molecules. The final purification step involves the separation, on the basis of size, of the desired toxin-hormone conjugates from toxin-toxin and hormone-hormone dimers; this is done by carrying out, in phosphate-buffered saline, Sephadex G100 chromotography.

### Use

The hybrid toxin-ligand molecules are administered to a mammal, e.g., a human, suffering from a medical disorder, e.g., cancer, characterized by the presence of a class of unwanted cells to which the ligand can selectively bind. The amount of hybrid molecule administered will vary with the type of disease, extensiveness of the disease, and size and species of the mammal suffering from the disease. Generally, amounts will be in the range of those used for other cytotoxic agents used in the treatment of cancer, although in certain instances lower amounts will be needed because of the specificity of the molecules.

The hybrid molecules can be administered using any conventional method; e.g., via injection, or via a timed-release implant. The hybrid proteins can be combined with any non-toxic, pharmaceutically-acceptable carrier substance.

In the case of MSH hybrids, topical creams can be used to kill primary melanoma cells, and injections or implants can be used to kill metastatic cells.

Estradiol hybrids, exhibiting binding specificity for certain breast cancers characterized by cells bearing estradiol receptors, can be used to treat primary and metastatic cells.

Other embodiments are within the following claims.

## Claims (Claims for the following Contracting State(s): BE, CH, DE, FR, GB, IT, LI, LU, NL, SE)

1. A targeted toxin molecule comprising an enzymatically active toxin moiety coupled to a cell specific ligand comprising a peptide, a proteinaceous growth factor or a modified steroid hormone, wherein the toxin moiety exhibits cytotoxic activity but fails to exhibit generalised eucaryotic cell binding and is encoded by a DNA sequence including a non-naturally occurring cysteine codon; and wherein the toxin moiety is coupled to the cell specific ligand via a disulfide linkage between the non naturally occurring cysteine residue and a site on the ligand.

2. A targeted toxin molecule as claimed in claim 1 wherein the toxin is diphtheria toxin, ricin or abrin.

3. A targeted toxin molecule as claimed in claim claim 1 or claim 2 wherein the ligand includes a sulphydryl group and the disulfide linkage is between the sulphydryl group and the non-naturally occurring cysteine residue of the toxin molecule.

4. A targeted toxin molecule as claimed in any one of claims 1 to 3 wherein the peptide is a hormone.

5. A targeted toxin molecule as claimed in any one of claims 1 to 3 wherein the proteinaceous growth factor is Interleukin I, Interleukin II, Interleukin III or B-cell growth factor.

6. A targeted toxin molecule as claimed in any one of claims 1 to 3 wherein the steroid hormone is estradiol.

7. A targeted toxin molecule comprising an enzymatically active toxin moiety coupled to a cell-specific ligand comprising a peptide, a proteinaceous growth factor or an antibody encoded by a DNA sequence including a non-naturally occurring cysteine codon, wherein the toxin moiety is coupled to the cell-specific ligand via a disulfide linkage between the non-naturally occurring cysteine residue and a site on the toxin molecule.

8. A targeted toxin molecule as claimed in claim 7, wherein the peptide is a hormone.

9. A targeted toxin molecule as claimed in claim 7, wherein the proteinaceous growth factor is Interleukin I, Interleukin II, Interleukin III or B-cell growth factor.

## Claims (Claims for the following Contracting State(s): AT)

1. A process for the preparation of a targeted toxin molecule comprising coupling an enzymatically active toxin moiety to a cell specific ligand comprising a peptide, a proteinaceous growth factor or a modified steroid hormone, wherein the toxin moiety exhibits cytotoxic activity but fails to exhibit generalised eucaryotic cell binding and is encoded by a DNA sequence including a non-naturally occurring cysteine codon; and wherein the toxin moiety is coupled to the cell specific ligand via a disulfide linkage between the non naturally occurring cysteine residue and a site on the ligand.

2. A process as claimed in claim 1 wherein the toxin is diphtheria toxin, ricin or abrin.

3. A process as claimed in claim 1 or claim 2 wherein the ligand includes a sulphydryl group and the disulfide linkage is between the sulphydryl group and the non-naturally occurring cysteine residue of the toxin molecule.

4. A process as claimed in any one of claims 1 to 3 wherein the peptide is a hormone.

5. A process as claimed in any one of claims 1 to 3 wherein the proteinaceous growth factor is Interleukin I, Interleukin II, Interleukin III or B-cell growth factor.

6. A process as claimed in any one of claims 1 to 3 wherein the steroid hormone is estradiol.

7. A process for the preparation of a targeted toxin molecule comprising coupling an enzymatically active toxin moiety to a cell-specific ligand comprising a peptide, a proteinaceous growth factor or an antibody encoded by a DNA sequence including a non-naturally occurring cysteine codon, wherein the toxin moiety is coupled to the cell-specific ligand via a disulfide linkage between the non-naturally occurring cysteine residue and a site on the toxin molecule.

8. A process as claimed in claim 7, wherein the peptide is a hormone.

9. A process as claimed in claim 7, wherein the proteinaceous growth factor is Interleukin I, Interleukin II, Interleukin III or B-cell growth factor.

## Patentansprüche (Patentansprüche für folgende(n) Vertragsstaat(en): BE, CH, DE, FR, GB, IT, LI, LU, NL, SE)

1. Zielgerichtetes Toxinmolekül mit einem enzymatisch aktiven Toxinanteil, der an einen zellspezifischen Liganden gekoppelt ist, welcher ein Peptid, einen proteinischen Wachstumsfaktor oder ein modifiziertes Steroidhormon aufweist, wobei der Toxinanteil cytotoxische Aktivität besitzt, aber keine generalisierte eukaryotische Zellbindung aufweist und durch eine DNA-Sequenz kodiert wird, die ein nicht natürlich vorkommendes Cysteinkodon enthält; und wobei der Toxinanteil an den zellspezifischen Liganden über eine Disulfidbindung zwischen dem nicht natürlich vorkommenden Cysteinrest und einer Stelle auf dem Liganden gekoppelt ist.

2. Zielgerichtetes Toxinmolekül nach Anspruch 1, wobei das Toxin Diphtherietoxin, Ricin oder Abrin ist.

3. Zielgerichtetes Toxinmolekül nach Anspruch 1 oder Anspruch 2, wobei der Ligand eine Sulfhydrylgruppe enthält und die Disulfidbindung zwischen der Sulfhydrylgruppe und dem nicht natürlich vorkommenden Cysteinrest des Toxinmoleküls besteht.

4. Zielgerichtetes Toxinmolekül nach einem der Ansprüche 1 bis 3, wobei das Peptid ein Hormon ist.

5. Zielgerichtetes Toxinmolekül nach einem der Ansprüche 1 bis 3, wobei der proteinische Wachstumsfaktor Interleukin I, Interleukin II, Interleukin III oder B-Zellen-Wachstumsfaktor ist.

6. Zielgerichtetes Toxinmolekül nach einem der Ansprüche 1 bis 3, wobei das Steroidhormon Östradiol ist.

7. Zielgerichtetes Toxinmolekül mit einem enzymatisch aktiven Toxinanteil, der an einen zellspezifischen Liganden gekoppelt ist, welcher ein Peptid, einen proteinischen Wachstumsfaktor oder einen Antikörper aufweist, der durch eine DNA-Sequenz kodiert wird, die ein nicht natürlich vorkommendes Cysteinkodon enthält, wobei der Toxinanteil an den zellspezifischen Liganden über eine Disulfidbindung zwischen dem nicht natürlich vorkommenden Cysteinrest und einer Stelle auf dem Toxinmolekül gekoppelt ist.

8. Zielgerichtetes Toxinmolekül nach Anspruch 7, wobei das Peptid ein Hormon ist.

9. Zielgerichtetes Toxinmolekül nach Anspruch 7, wobei der proteinische Wachstumsfaktor Interleukin I, Interleukin II, Interleukin III oder B-Zellen-Wachstumsfaktor ist.

## Patentansprüche (Patentansprüche für folgende(n) Vertragsstaat(en): AT)

1. Verfahren zur Herstellung eines zielgerichteten Toxinmoleküls, bestehend aus dem Koppeln eines enzymatisch aktiven Toxinanteils an einen zellspezifischen Liganden, welcher ein Peptid, einen proteinischen Wachstumsfaktor oder ein modifiziertes Steroidhormon aufweist, wobei der Toxinanteil cytotoxische Aktivität besitzt, aber keine generalisierte eukaryotische Zellbindung aufweist und durch eine DNA-Sequenz kodiert wird, die ein nicht natürlich vorkommendes Cysteinkodon enthält; und wobei der Toxinanteil an den zellspezifischen Liganden über eine Disulfidbindung zwischen dem nicht natürlich vorkommenden Cysteinrest und einer Stelle auf dem Liganden gekoppelt ist.

2. Verfahren nach Anspruch 1, wobei das Toxin Diphtherietoxin, Ricin oder Abrin ist.

3. Verfahren nach Anspruch 1 oder Anspruch 2, wobei der Ligand eine Sulfhydrylgruppe enthält und die Disulfidbindung zwischen der Sulfhydrylgruppe und dem nicht natürlich vorkommenden Cysteinrest des Toxinmoleküls besteht.

4. Verfahren nach einem der Ansprüche 1 bis 3, wobei das Peptid ein Hormon ist.

5. Verfahren nach einem der Ansprüche 1 bis 3, wobei der proteinische Wachstumsfaktor Interleukin I, Interleukin II, Interleukin III oder B-Zellen-Wachstumsfaktor ist.

6. Verfahren nach einem der Ansprüche 1 bis 3, wobei das Steroidhormon Östradiol ist.

7. Verfahren zur Herstellung eines zielgerichteten Toxinmoleküls, bestehend aus dem Koppeln eines enzymatisch aktiven Toxinanteils an einen zellspezifischen Liganden, welcher ein Peptid, einen proteinischen Wachstumsfaktor oder einen Antikörper aufweist, der durch eine DNA-Sequenz kodiert wird, die ein nicht natürlich vorkommendes Cysteinkodon enthält, wobei der Toxinanteil an den zellspezifischen Liganden über eine Disulfidbindung zwischen dem nicht natürlich vorkommenden Cysteinrest und einer Stelle auf dem Toxinmolekül gekoppelt ist.

8. Verfahren nach Anspruch 7, wobei das Peptid ein Hormon ist.

9. Verfahren nach Anspruch 7, wobei der proteinische Wachstumsfaktor Interleukin I, Interleukin II, Interleukin III oder B-Zellen-Wachstumsfaktor ist.

## Revendications (Revendications pour l'(les) Etat(s) contractant(s) suivant(s): BE, CH, DE, FR, GB, IT, LI, LU, NL, SE)

1. Molécule de toxine dirigée comprenant une partie toxine enzymatiquement active couplée à un ligand cellulaire spécifique comprenant un peptide, un facteur de croissance protéique ou une hormone stéroïde modifiée, où la partie toxine exhibe une activité cytotoxique, mais ne présente pas une liaison généralisée aux cellules eucaryotes et est codée par une séquence d'ADN contenant un codon cystéine non naturel; et où la partie toxine est couplée au ligand cellulaire spécifique par un pont disulfure entre le résidu cystéine non naturel et un site sur le ligand.

2. Molécule de toxine dirigée suivant la revendication 1, où la toxine est la toxine diphtérique, la ricine ou l'abrine.

3. Molécule de toxine dirigée suivant la revendication 1 ou 2, où le ligand comprend un radical sulfhydryle et le lien disulfure se fait entre le radical sulfhydryle et le résidu cystéine non naturel de la molécule de toxine.

4. Molécule de toxine dirigée suivant l'une quelconque des revendications 1 à 3, où le peptide est une hormone.

5. Molécule de toxine dirigée suivant l'une quelconque des revendications 1 à 3, où le facteur de croissance protéique est l'interleukine I, l'interleukine II, l'interleukine III ou un facteur de croissance des cellules B.

6. Molécule de toxine dirigée suivant l'une quelconque des revendications 1 à 3, où l'hormone stéroïde est l'oestradiol.

7. Molécule de toxine dirigée comprenant une partie toxine enzymatiquement active couplée à un ligand cellulaire spécifique comprenant un peptide, un facteur de croissance protéique ou un anticorps codé par une séquence d'ADN contenant un codon cystéine non naturel, où la partie toxine est couplée au ligand cellulaire spécifique par un pont disulfure entre le résidu cystéine non naturel et un site de la molécule de toxine.

8. Molécule de toxine dirigée suivant la revendication 7, où le peptide est une hormone.

9. Molécule de toxine dirigée suivant la revendication 7, où le facteur de croissance protéique est l'interleukine I, l'interleukine II, l'interleukine III ou un facteur de croissance des cellules B.

## Revendications (Revendications pour l'(les) Etat(s) contractant(s) suivant(s): AT)

1. Procédé pour la préparation d'une molécule de toxine dirigée comprenant le couplage d'une partie toxine enzymatiquement active à un ligand cellulaire spécifique comprenant un peptide, un facteur de croissance protéique ou une hormone stéroïde modifiée, où la partie toxine exhibe une activité cytotoxique, mais ne montre pas de liaison généralisée aux cellules eucaryotes et est codée par une séquence d'ADN contenant un codon cystéine non naturel, et où la partie toxine est couplée au ligand cellulaire spécifique par un pont disulfure entre le résidu cystéine non naturel et un site du ligand.

2. Procédé suivant la revendication 1, où la toxine est la toxine diphtérique, la ricine ou l'abrine.

3. Procédé suivant la revendication 1 ou 2, où le ligand comprend un radical sulfhydryle et le pont disulfure se fait entre le radical sulfhydryle et le résidu cystéine non naturel de la molécule de toxine.

4. Procédé suivant l'une quelconque des revendications 1 à 3, où le peptide est une hormone.

5. Procédé suivant l'une quelconque des revendications 1 à 3, où le facteur de croissance protéique est l'interleukine I, l'interleukine II, l'interleukine III ou un facteur de croissance des cellules B.

6. Procédé suivant l'une quelconque des revendications 1 à 3, où l'hormone stéroïde est l'oestradiol.

7. Procédé pour la préparation d'une molécule de toxine dirigée comprenant le couplage d'une partie toxine enzymatiquement active à un ligand cellulaire spécifique comprenant un peptide, un facteur de croissance protéique ou un anticorps codé par une séquence d'ADN contenant un codon cystéine non naturel, où la partie toxine est couplée au ligand cellulaire spécifique par un pont disulfure entre le résidu cystéine non naturel et un site de la molécule de toxine.

8. Procédé suivant la revendication 7, où le peptide est une hormone.

9. Procédé suivant la revendication 7, où le facteur de croissance protéique est l'interleukine I, l'interleukine II, l'interleukine III ou un facteur de croissance des cellules B.
